# EUROPEAN PATENT APPLICATION

(11) **EP 4 818 700 A1**
(43) Date of publication of application: **07.10.2026**
(21) Application number: 25167658.1
(22) Date of filing: 01.04.2025
(51) Int. Cl.: G01N 33/574, G01N 33/68

(54) **MELANOMA BIOMARKER**

(71) Applicant: Universität Zürich, 8006 Zürich (CH)
(72) Inventor: SCHARL, Michael, 8091 Zürich (CH); MORSY, Yasser, 8091 Zürich (CH); LEVESQUE, Mitchell, 8091 Zürich (CH)
(74) Representative: Schulz Junghans Patentanwälte PartGmbB

(57) **Abstract**

The invention relates to methods of detecting whether melanoma is present in a subject comprising measuring metabolite concentrations in a sample obtained from the subject.

## Description

### Field

The present invention relates to metabolite biomarkers useful for detecting the presence of melanoma in a subject.

### Background

Melanoma is one of the deadliest cancers, and biomarkers for accurate and early diagnosis are urgently needed. Currently available biomarkers can presently support histopathologic diagnosis and disease stating staging, assignment of prognosis and monitoring the therapeutic efficacy of specific therapies. Among those biomarkers are approaches based on the detection of circulating tumour cells, serum nucleic acid levels, gene and protein expression levels and patterns as well as genetic variations (Deacon, 2021). However, reliable biomarkers for identifying early stage disease, facilitating diagnosis and disease monitoring of malignant melanoma at all disease stages are not available. For example, serum levels of lactate dehydrogenase or S-100 protein are particularly useful only for monitoring disease course or recurrence, but not for diagnostic purposes (Weide, 2012; Gershenwald, 2017). Highly accurate and easily measurable biomarkers are desirable in daily clinical practice, as early detection and treatment of melanoma is critical to improve the prognosis for the patients (Baade, 2020)

Notably, current knowledge about the serum metabolome in patients with melanoma is scarce. Most studies addressing the role of the metabolome in melanoma focused either on melanoma cells or melanoma tissue (Weber 2021; Taylor 2020; Du, 2020; Kim, 2017; Shi, 2017) or even on the faecal metabolome (Frankel 2017; Bui, 2021; Vandoni, 2023). One study performed a small, targeted analysis of serum metabolites and observed a significant difference between patients with melanoma and healthy individuals, identifying several potential metabolite biomarkers when analyzing 181 metabolites in the serum of 26 patients with melanoma stage III or IV (Bayci, 2018). However, the study the study was small and lacked validation of candidate biomarkers. It also included only late disease stage III or IV patients, and it remains unknown if early stage disease is characterized by a distinct systemic metabolite profile.

Based on the above-mentioned state of the art, the objective of the present invention is to provide means and methods to detect the presence of melanoma in a subject. This objective is attained by the subject-matter of the independent claims of the present specification, with further advantageous embodiments described in the dependent claims, examples, figures and general description of this specification.

### Summary of the Invention

A non-targeted serum metabolome analysis performed by the inventors identified powerful biomarkers that indicate the presence of melanoma at any stage of the disease progression, facilitating diagnostic and clinical status tracking of early disease in blood samples for the first time.

A first aspect of the invention relates to methods for detecting the presence of a melanoma in a subject comprising measuring, in a sample having been previously obtained from said subject, a concentration value of at least one metabolite selected the list disclosed in Table 2. In particular embodiments, the metabolite concentration value is obtained for at least one of the following list of metabolites providing highly accurate patient classification:
- muramic acid,
- N-(1-deoxy-1-fructosyl)leucine,
- N-(1-deoxy-1-fructosyl)valine,
- N-(1-deoxy-1-fructosyl)methionine,
- 1-[(5-amino-5-carboxypentyl)amino]-1-deoxyfructose, and/or
- Dihydrolipoate,
- N-(1-deoxy-1-fructosyl)-glycine,
- N-(1-deoxy-1-fructosyl)-histidine,
- N-(1-deoxy-1-fructosyl)-leucine,
- distichonic acid A,
- hydroxypyruvate,
- toluene-cis-dihydrodiol
- acetyl-leucine.

In particular embodiments of the method, the measurement step comprises determining a concentration value of muramic acid (or a derivative thereof) in a patent sample, particularly a blood sample such as plasma or serum.

In particular embodiments, the method further comprises steps of comparing the metabolite concentration value from the measurement to a reference sample or threshold representing the level of the metabolite in healthy human controls. In further particular embodiments, the method comprises assigning the presence of melanoma to the subject depending on whether the metabolite concentration value reflects that of patients and not healthy controls (see Table 2) with reference to said threshold.

The concentration value of the metabolite can be measured by methods known the skilled person, such as High performance liquid chromatography (HPLC) or gas chromatography (GC) coupled to a suitable detection method such as, for example, UV/VIS absorption, nuclear magnetic resonance (NMR), or mass spectrometry (MS), or targeted enzyme-linked immune-sorbent assay (ELISA), or a radio immune assay.

The invention further encompasses kits, biomarker compositions, and diagnostic systems based on measuring the concentration value of biomarker metabolites according to the first aspect of the invention.

The invention further relates to methods of diagnosing a subject with melanoma, methods for providing diagnostic information to a subject suspected of having melanoma, and methods of evaluating a cancer patient based on metabolite concentration value.

The invention further encompasses methods of treating a patient with a gold-standard melanoma therapy such as a checkpoint inhibition agent, or a BRAF or MEK inhibitor, when the method is combined with melanoma staging evaluation indicated advanced disease. The method comprises a step of measuring a biomarker metabolite according to the invention in the sample, prior to melanoma staging evaluation, or other clinical evaluation steps such as BRAF or MEK gene expression assays.

### Terms and definitions

### General

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *muramic acid* in the context of the present specification relates to the ether of lactic acid and glucosamine with the formula C₉H₁₇NO₇. Muramic acid is a mixture of alpha- beta-anomers, also sometimes referred to as muramate, or 2-amino-3-O-[(R)-1-carboxyethyl]-2-deoxy-D-glucopyranose. The molecular weight is 251.23 g / mol and it is assigned Cas number is 1114-41-6, and human metabolite database (HMDB) identifier HMDB0003254.

The term N-*(1-deoxy-1-fructosyl)leucine* in the context of the present specification relates to C₁₂H₂₃NO₇, with the HMDB identifier HMDB0037840, associated with Cas no. 34393-18-5. The molecular weight is 293.31 g / mol, and the metabolite can also be referred to as (2S)-4-methyl-2-({[(3S,4S,5R)-2,3,4-trihydroxy-5-(hydroxymethyl)oxolan-2-yl]methyl}amino)pentanoic acid.

The term *N-(1-deoxy-1-fructosyl)valine* in the context of the present specification relates to the metabolite of formula C₁₂H₂₁NO₇, sometimes referred to herein by the HMDB identifier HMDB0037844. The molecular weight is 279.29 g / mol. The metabolite is sometimes referred to as (2S)-3-methyl-2-({[(3S,4S,5R)-2,3,4-trihydroxy-5-(hydroxymethyl)oxolan-2-yl]methyl}amino)butanoic acid.

The term *N-(1-deoxy-1-fructosyl)methionine* in the context of the present specification relates to the metabolite of formula C₁₁H₂₁NO₇S, sometimes referred to herein by the HMDB identifier HMDB0037841. The molecular weight is 311.35 g / mol. The metabolite is sometimes referred to as (2S)-3-methyl-2-({[(3S,4S,5R)-2,3,4-trihydroxy-5-(hydroxymethyl)oxolan-2-yl]methyl}amino)butanoic acid.

The term *1-[(5-amino-5-carboxypentyl)amino]-1-deoxyfructose* in the context of the present specification relates to the metabolite of formula C₁₂H₂₄N₂O₇, sometimes referred to herein by the HMDB identifier HMDB0034879. The molecular weight is 308.32 g / mol. The metabolite is sometimes referred to as 2-amino-6-({[2,3,4-trihydroxy-5-(hydroxymethyl)oxolan-2-yl]methyl}amino)hexanoic acid, and is assigned the Cas no. 21291-40-7.

The term dihydrolipoate in the context of the present specification relates to the metabolite of formula C₈H₁₆O₂S₂, sometimes referred to herein by the HMDB identifier HMDB0012210. The molecular weight is 208.34 g / mol. The metabolite is sometimes referred to as 6,8-disulfanyloctanoic acid, or dihydrolipoic acid, and is assigned the Cas no. 462-20-4.

The term *melanoma* in the context of the present specification relates to the form of cancer derived from a melanocyte in skin or pigmented tissues. The term encompasses various subclasses such as, but not limited to, superficial spreading melanoma, nodular melanoma, acral-lentiginous melanoma, lentigo maligna melanoma, amelanotic melanoma, and desmoplastic melanoma. Melanoma commonly affects skin, but can arise from internal tissues or the eyes. The term encompasses stages of increasing severe disease from a tumour of up to 2mm thickness (stage I) through disseminated disease, i.e. metastatic melanoma that has spread to secondary organs (stage IV).

The term *concentration value* of a metabolite in the context of the present specification refers to relative or absolute values reflecting abundance of the metabolite in a subject's sample. The term encompasses for example, a quantitative measurement of absolute concentration measured with reference to an appropriate chemical standard. Alternatively, a concentration value determined for a patient sample can be expressed with reference to a control sample, for example a concentration value can be expressed as a fold-change compared to a control. The term encompasses either raw data from a measurement device, or the output of bioinformatic methods to normalise raw mass spectrometry (MS), or nuclear magnetic resonance (NMR) measurement data which allow the same metabolites measured within an array of different spectra to be compared (for example between controls, samples, or standards) to improve the reliability and interpretability of downstream statistical analysis and clinical classifications. An example of normalised intensity value for a biomarker metabolite where the concentration value has been determined by mass spectrometry, can be expressed for example, in terms of its relative abundance to other metabolites measured in the same sample, or a reference spectrum (e.g. constant sum normalisation, probabilistic quotient normalisation to a reference spectrum, or vector length normalisation or internal standard normalisation towards a selected internal reference metabolite).

### Cell Biology, diagnostic method inventions: Markers, ligands

In the present specification, the level of metabolite may be assayed via techniques such as nuclear magnetic resonance, mass spectrometry, flow cytometry, ELISPOT, enzyme-linked immunosorbance assay (ELISA) or multiplex analyses.

If not specified more narrowly in the *Detailed Description of the Invention,* reference to molecular probes, binders and ligands encompasses antibodies, antibody-like molecules and aptamers is defined in the following paragraphs.

The term *molecular probe* in the context of the present specification relates to a specific ligand, particularly an antibody, antibody fragment, an antibody-like molecule or aptamer, more particularly an antibody or antibody fragment, that can bind to the target biomarker metabolite molecule, human Ig, with a dissociation constant of ≤10⁻⁷ mol/l, particularly ≤10⁻⁹ mol/l. The molecular probe comprises a detectable label such as a particle, bead, dye, or enzyme, or a genetically engineered tags such as poly His-tag, T7-tag, and E-tag. Such tags are commonly used as epitopes of secondary antibodies for monoclonal antibodies, or antibody fragments, because they can be genetically incorporated into antibody genes to provide a 1:1 signal without disturbing the tertiary structure and activity of the binding domain through chemical labelling protocols.

The term *specific binding* in the context of the present invention refers to a property of ligands that bind to their target with a certain affinity and target specificity. The affinity of such a ligand is indicated by the dissociation constant of the ligand. A specifically reactive ligand has a dissociation constant of ≤ 10⁻⁸mol/L (particularly ≤ 10⁻⁹mol/L) when binding to its target, but a dissociation constant at least three orders of magnitude higher in its interaction with a molecule having a globally similar chemical composition as the target, but a different three-dimensional structure.

In the context of the present specification, the term *dissociation constant (K_{D})* is used in its meaning known in the art of chemistry and physics; it refers to an equilibrium constant that measures the propensity of a complex composed of [in most cases, two] different components to dissociate reversibly into its constituent components. The complex can be e.g. an antibody-antigen complex AbAg composed of antibody Ab and antigen Ag. K_{D} is expressed in molar concentration [mol/I] and corresponds to the concentration of [Ab] at which half of the binding sites of [Ag] are occupied, in other words, the concentration of unbound [Ab] equals the concentration of the [AbAg] complex. The dissociation constant can be calculated according to the following formula: ${K}_{D} = \frac{\left[Ab\right] ∗ \left[Ag\right]}{\left[AbAg\right]}$ [Ab]: concentration of antibody; [Ag]: concentration of antigen; [AbAg]: concentration of antibody-antigen complex

In the context of the present specification, the terms *off-rate* (K_{off};[1/sec]) and *on-rate* (Kₒₙ; [L/(sec*mol)]) are used in their meaning known in the art of chemistry and physics; they refer to a rate constant that measures the dissociation (K_{off}) or association (Kₒₙ) of an antibody with its target antigen. K_{off} and Kₒₙ can be experimentally determined using methods well established in the art. A method for determining the K_{off} and Kₒₙ of an antibody employs surface plasmon resonance. This is the principle behind biosensor systems such as the Biacore^{®} or the ProteOn^{®} system. They can also be used to determine the dissociation constant K_{D} by using the following formula: ${K}_{D} = \frac{\left[{K}_{off}\right]}{\left[{K}_{on}\right]}$

The natural upper limit for the on-rate Kₒₙ is 10⁹ L/(sec*mol).

In the context of the present specification, the term *antibody* refers to whole antibodies including but not limited to immunoglobulin type G (IgG), type A (IgA), type D (IgD), type E (IgE) or type M (IgM), any antigen-binding fragment or single chains thereof and related or derived constructs. A whole antibody is a glycoprotein comprising at least two heavy (H) chains and two light (L) chains inter-connected by disulfide bonds. Each heavy chain is comprised of a heavy chain variable region (V_{H}) and a heavy chain constant region (C_{H}). The heavy chain constant region of IgG is comprised of three domains, C_{H}1, C_{H}2 and C_{H}3. Each light chain is comprised of a light chain variable region (abbreviated herein as V_{L}) and a light chain constant region (C_{L}). The light chain constant region is comprised of one domain, C_{L}. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component of the classical complement system. Similarly, the term encompasses a so-called nanobody or single domain antibody, an antibody fragment consisting of a single monomeric variable antibody domain.

The term *antibody-like molecule* in the context of the present specification refers to a molecule capable of specific binding to another molecule or target with high affinity / a Kd ≤ 10⁻⁷ mol/L (particularly ≤ 10⁻⁹mol/L). An antibody-like molecule binds to its target similarly to the specific binding of an antibody. The term *antibody-like molecule* encompasses a repeat protein, such as a designed ankyrin repeat protein (Molecular Partners, Zürich), an engineered antibody mimetic protein exhibiting highly specific and high-affinity target protein binding (see US2012142611, US2016250341, US2016075767 and US2015368302). The term antibody-like molecule further encompasses, but is not limited to, a polypeptide derived from armadillo repeat proteins, a polypeptide derived from leucine-rich repeat proteins and a polypeptide derived from tetratricopeptide repeat proteins. The term *antibody-like molecule* further encompasses a specifically binding polypeptide derived from a protein A domain, a fibronectin domain FN3, a consensus fibronectin domain, a lipocalin (see Skerra, Biochim. Biophys. Acta 2000, 1482(1-2):337-50), a polypeptide derived from a Zinc finger protein (see Kwan et al. Structure 2003, 11(7):803-813), a Src homology domain 2 (SH2) or Src homology domain 3 (SH3), a PDZ domain, a gamma-crystallin, ubiquitin, a cysteine knot polypeptide or a knottin, cystatin, Sac7d, a triple helix coiled coil (also known as alphabodies), a Kunitz domain or a Kunitz-type protease inhibitor and a carbohydrate binding module 32-2. The term *antibody-like molecule* further encompasses a *humanized camelid antibody.* The term *antibody-like molecule* similarly encompasses an scFv fragment. Fab fragments and scFv are desirable reagents for ELISA because the original affinity and specificity of the original IgG molecule are maintained, and background from Fc portions is minimised.

The term *aptamer* relates to an oligonucleotide or peptide molecule that binds to a specific target molecule. Aptamers can be created by selecting them from a large random sequence pool. Nucleic acid aptamers can be generated through repeated rounds of in-vitro selection or equivalently, SELEX (systematic evolution of ligands by exponential enrichment) to bind to molecular targets such as small molecules, proteins or nucleic acids through non-covalent interactions. Aptamers offer molecular recognition properties that rival that of antibodies.

The term *Detectable Label* or *detectable marker* the context of the present specification relates to a molecule, with distinctive properties that is conjugated or attached to a carrier molecule, such as a ligand (molecular probe), more specifically the ligand being an antibody, to facilitate the detection and quantification of the ligand's metabolite target in a biological sample. The label, or marker allows visual or instrumental identification of the presence (qualitative measurement), or concentration (quantitative measurement) of the target molecule. Common types of detectable labels used in procedures such as ELISA and other diagnostic methods include enzymes, fluorescent molecules, fluorescent dye, radioactive isotopes, colloidal gold particles, and biotin, among others. These labels emit signals or exhibit distinct properties that can be measured, or visualized, to determine the presence and/or quantity of the target molecule in the sample.

The term *fluorescent dye* in the context of the present specification relates to a small molecule capable of fluorescence in the visible, or near infrared spectrum. Examples of fluorescent labels or labels presenting a visible colour include, without being restricted to, fluorescein isothiocyanate (FITC), rhodamine, allophycocyanin (APC), peridinin chlorophyll (PerCP), phycoerythrin (PE), Alexa Fluors (Life Technologies, Carlsbad, CA, USA), dylight fluors (Thermo Fisher Scientific, Waltham, MA, USA) ATTO Dyes (ATTO-TEC GmbH, Siegen, Germany), BODIPY Dyes (4,4-difluoro-4-bora-3a,4a-diaza-s-indacene based dyes).

### Melanoma therapy

In the context of the present specification, the term *checkpoint inhibitory agent* or *checkpoint inhibitory antibody* is meant to encompass an agent, particularly an antibody (or antibody-like molecule) capable of disrupting the signal cascade leading to T cell inhibition after T cell activation as part of what is known in the art the immune checkpoint mechanism. Non-limiting examples of a *checkpoint inhibitory agent* or *checkpoint inhibitory antibody* include antibodies to CTLA-4 (Uniprot P16410) such as ipilimumab (Yervoy; CAS No. 477202-00-9), or antibodies to PD-1 (Uniprot Q15116) or to PD-L1 (Uniprot Q9NZQ7), B7H3 (CD276; Uniprot Q5ZPR3), exemplified by the clinically available antibody drugs nivolumab (Bristol-Myers Squibb; CAS No 946414-94-4), pembrolizumab (Merck Inc.; CAS No. 1374853-91-4), pidilizumab (CAS No. 1036730-42-3), atezolizumab (Roche AG; CAS No. 1380723-44-3), and Avelumab (Merck KGaA; CAS No. 1537032-82-8), Durvalumab (Astra Zenaca, CAS No. 1428935-60-7), and Cemiplimab (Sanofi; CAS No. 1801342-60-8).

In the context of the present specification, the term *BRAF inhibitory agent* refers to an agent which inhibits the action of the BRAF protein, examples include vemurafenib (Cas no. 918594-65-1), dabrafenib (Cas no. 1195765-45-7), and encorafenib (Cas no. 1269440-17-6).

In the context of the present specification, the term *MEK inhibitory agent* refers to an agent which inhibits the action of the MEK protein, examples include trametinib (Cas no. 971700-17-3), cobimetinib (Cas no. 934660-93-2), and binimetinib (Cas no. 606143-89-9).

As used herein, the term *treating* or *treatment* of melanoma refers in one embodiment to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavouring agents, dyes, and the like and combinations thereof, as would be known to those skilled in the art (see, for example, Remington: the Science and Practice of Pharmacy, ISBN 0857110624). The invention also encompasses nanoparticles, liposomes, or cellular carriers within the meaning of *pharmaceutically acceptable carrier.*

### Detailed Description of the Invention

The inventors performed an untargeted metabolomics analysis in an exploratory cohort of 87 patients with melanoma, in an independent validation cohort of 37 additional patients with melanoma featuring late-stage tumours, as well as in 18 healthy control individuals. The serum metabolome composition of patients with melanoma was strikingly different from that of healthy control individuals. The most abundant metabolites belong to lipids, fatty acyls, and organic acids. The inventors validated six serum metabolites that are able to predict the presence of melanoma with an AUC > 0.9544 in advanced stage melanoma, providing highly important diagnostic information. Importantly, the AUC of biomarkers such as muramic acid was highly informative even in very early stage I (n=22) and stage II (n=67) melanoma patients, independent of the age of the respective individuals. The following aspects of the invention provide new and powerful biomarkers for diagnostic and theragnostic clinical practice.

A first aspect of the invention relates to a method to detect melanoma in a subject, comprising a step of measuring a concentration value of at least one metabolite listed in Table 2 in a sample having been previously obtained from said subject. All metabolites in Table 2 discriminate between samples obtained from patients with melanoma and those of healthy controls, with high sensitivity and specificity.

In some embodiments, a metabolite concentration value is determined from one or more of the following highly discriminating biomarkers:
- muramic acid (C₉H₁₇NO₇),
- N-(1-deoxy-1-fructosyl)leucine (C₁₂H₂₃NO₇),
- N-(1-deoxy-1-fructosyl)valine (C₁₂H₂₃NO₇),
- N-(1-deoxy-1-fructosyl)methionine (C₁₁H₂₁NO₇S),
- 1-[(5-amino-5-carboxypentyl)amino]-1-deoxyfructose (C₁₂H₂₄N₂O₇), and
- dihydrolipoate (C₈H₁₆O₂S₂),
- N-(1-deoxy-1-fructosyl)-glycine,
- N-(1-deoxy-1-fructosyl)-histidine,
- N-(1-deoxy-1-fructosyl)-leucine,
- distichonic acid A,
- hydroxypyruvate,
- toluene-cis-dihydrodiol
- acetyl-leucine

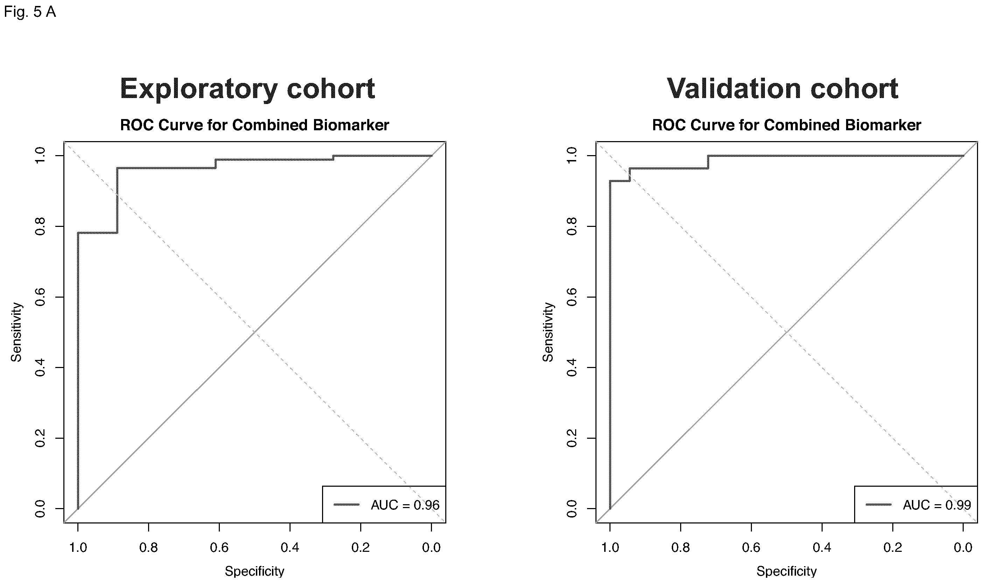

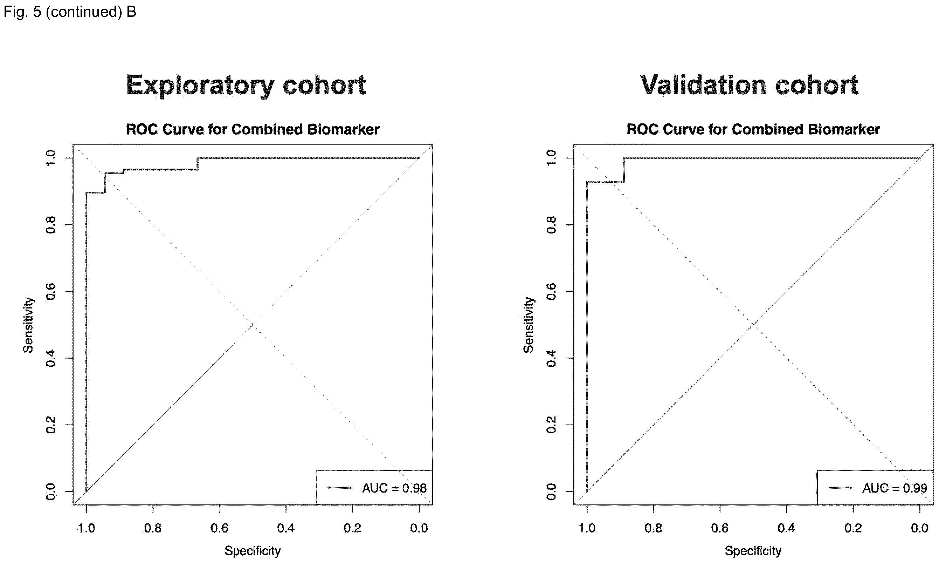

Optionally, a metabolite concentration of 2, 3, 4, 5, 6, 10, or more metabolite values are combined to discriminate between patient and control samples, as shown in Figure. 5.

In particular embodiments, the method comprises obtaining a concentration value for at least one of the following highly informative biomarkers that are increased in samples obtained from melanoma patients compared to healthy controls at all stages of disease:
- muramic acid (C₉H₁₇NO₇),
- N-(1-deoxy-1-fructosyl)leucine (C₁₂H₂₃NO₇),
- N-(1-deoxy-1-fructosyl)valine (C₁₂H₂₃NO₇),
- N-(1-deoxy-1-fructosyl)methionine (C₁₁H₂₁NO₇S),
- 1-[(5-amino-5-carboxypentyl)amino]-1-deoxyfructose (C₁₂H₂₄N₂O₇), and
- dihydrolipoate (C₈H₁₆O₂S₂).

Each of the above biomarkers in Table 2, and particular the 6 above provide a robust receiver operating curve (ROC) area under the curve (AUC) value indicating accurate distinction between melanoma patient and control samples in the Examples shown herein.

In other embodiments, the method comprises measuring at least 2, 3, 4, 5, or 6 metabolite concentration values. In certain embodiments, the method comprises measuring the metabolite concentration value of all 6 metabolites listed above.

In some embodiments of the method to detect melanoma according to the invention, the method comprises measuring a single metabolite biomarker concentration value from the list above. In particular embodiments of the method it comprises measuring a metabolite value of muramic acid. In particular embodiments, muramic acid is the only metabolite for which the concentration value is determined.

In certain embodiments, the method to detect melanoma further comprises comparing the concentration value to a threshold or a reference sample. Preferably, the reference sample is representative of one or more matched type samples for a subject who does not have melanoma. In certain embodiments, the reference sample is a control sample obtained from one or more healthy human subjects.

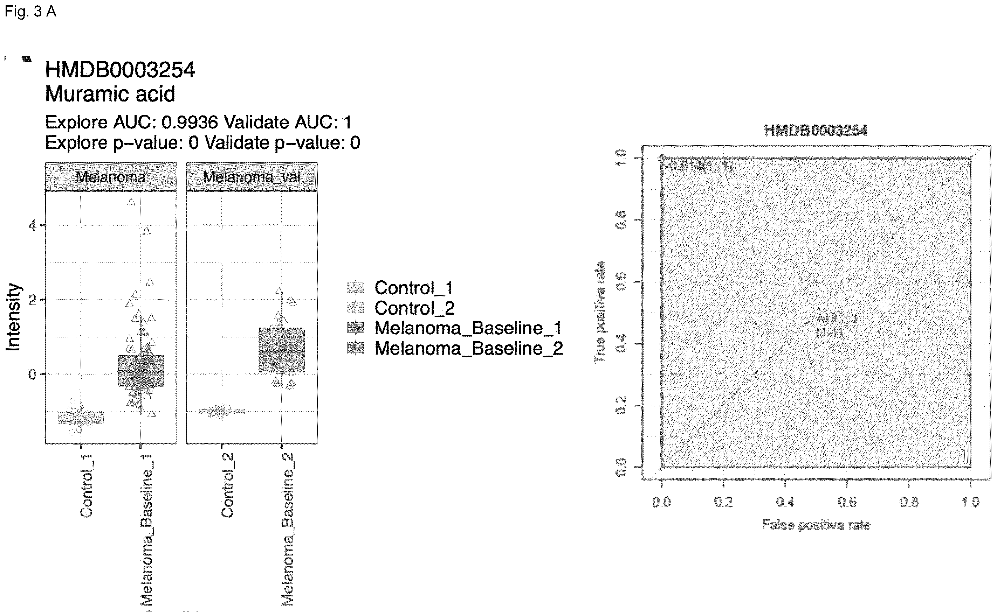

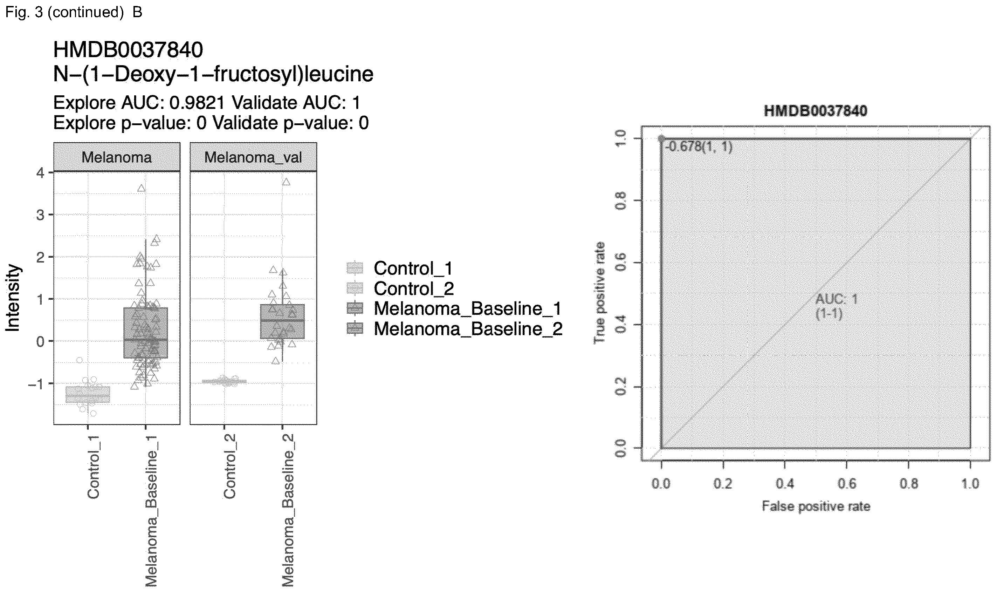

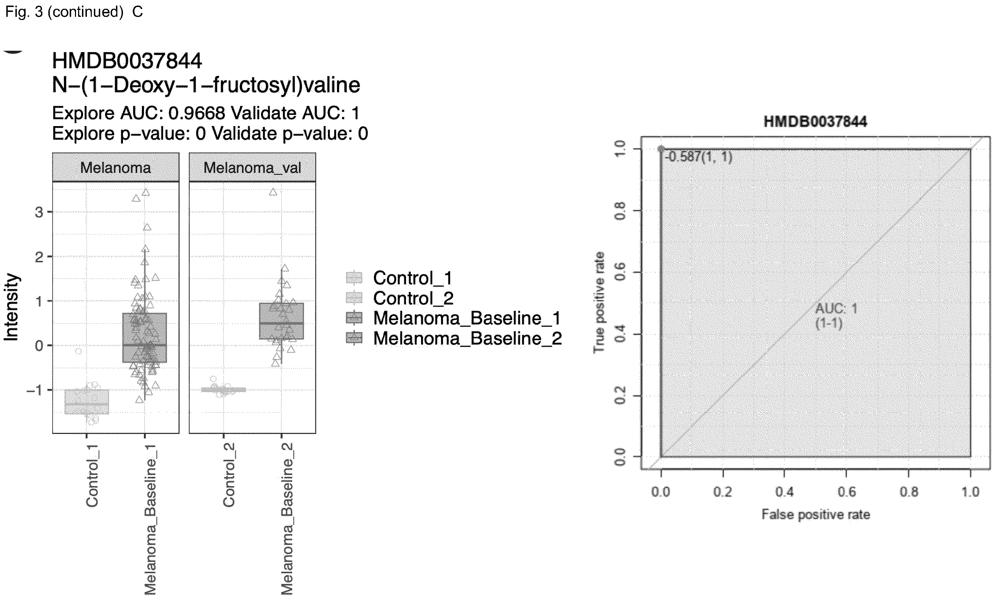

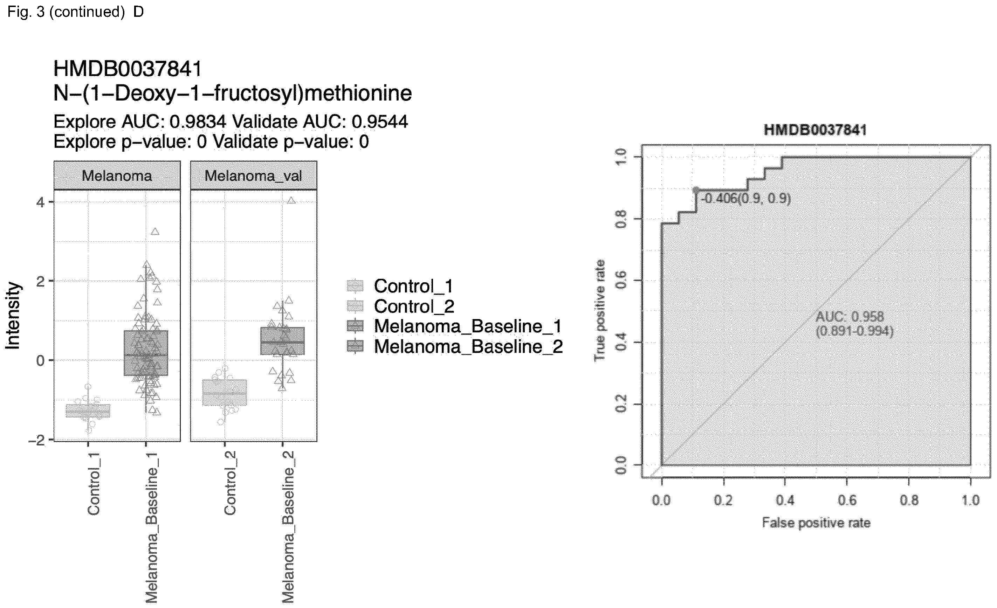

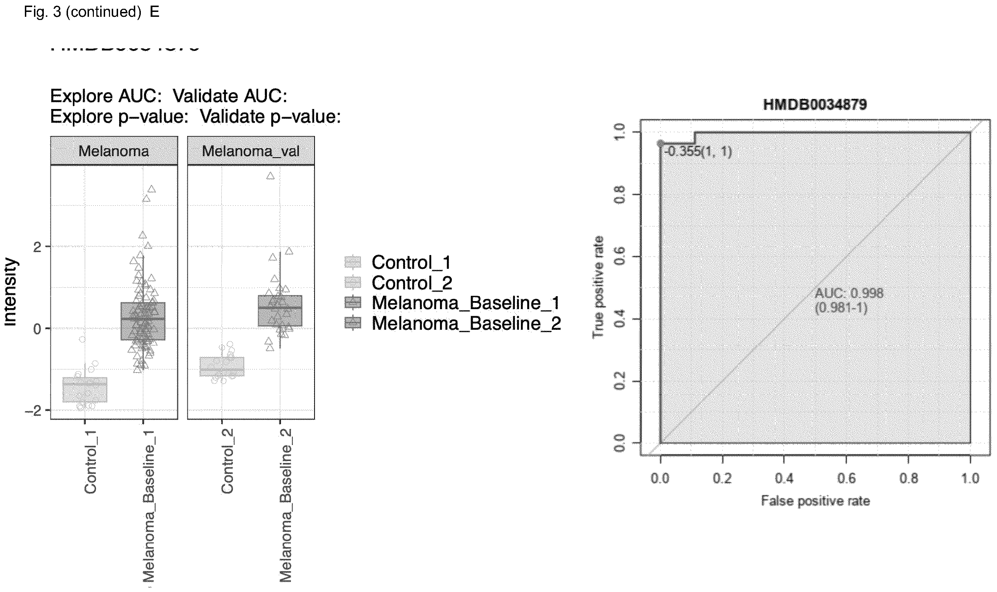

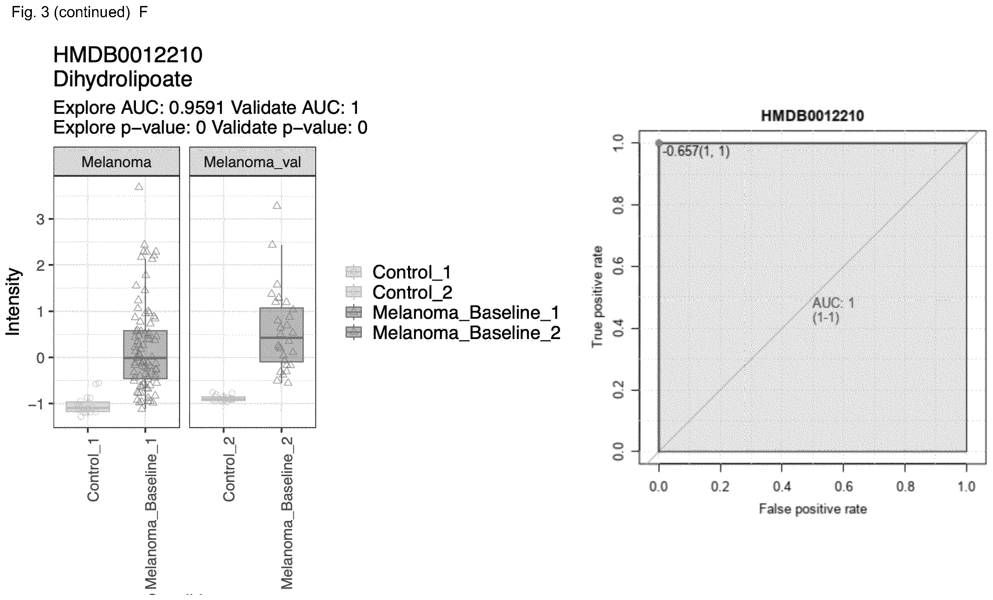

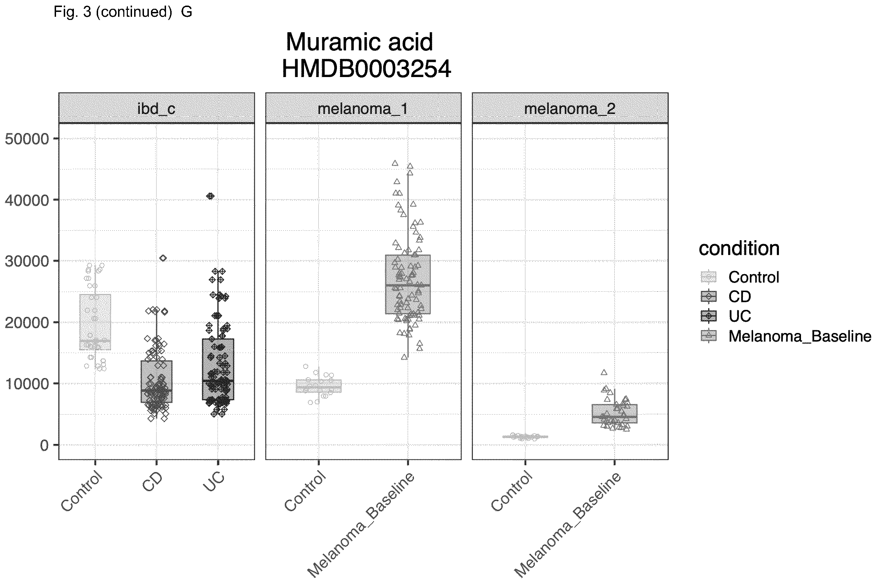

In certain embodiments, the method to detect melanoma further comprises assigned the patient to one of two classes. The classes indicate the presence of melanoma or not based on whether they are upregulated or downregulated in the melanoma patient cohort, see Table 2. The six metabolites discussed in Figure 3 including muramic acid are all significantly higher in patients versus control samples. Thus, a first class is assigned indicating the presence of melanoma to the subject if the metabolite concentration value is significantly increased in the sample relative to the threshold or a reference sample. A second class is assigned indicating the presence of melanoma to the subject if the metabolite concentration value is significantly decreased in the sample relative to the threshold or a reference sample. Optionally, the concentration values are entered into a classification algorithm that assigns an outcome class on the basis of two or more metabolite levels.

To quantify the amino acid or sugar derivatives found to be indicative of melanoma according to the invention in a blood sample, LC-MS/MS is a particularly useful method due to its high sensitivity and specificity, allowing for simultaneous detection of multiple analytes with minimal sample volume; derivatization is often used to enhance ionization or separation. GC-MS is also suitable, particularly for volatile or derivatized sugar compounds, though it requires more extensive sample preparation. HPLC with UV or fluorescence detection is a cost-effective alternative, especially when combined with derivatization agents such as OPA or FMOC for amino acids, and PMP for sugars. Capillary electrophoresis-mass spec (CE-MS) offers excellent resolution for small, charged molecules like amino acids, though it has lower throughput and higher variability than LC-MS/MS. Enzymatic or colorimetric assays can be used for routine quantification of common compounds but lack the specificity needed for modified derivatives.

In certain embodiments of the method to detect melanoma according to the invention, the concentration value of the metabolite is measured by HPLC or GC coupled to a suitable detection method such as, for example, UV/VIS absorption, nuclear magnetic resonance (NMR), or mass spectrometry (MS), by targeted enzyme-linked immune-sorbent assay (ELISA), or by a radio immune assay. In particular embodiments of the method the level of the metabolite is measured by liquid chromatography combined with mass spectrometry (LC/MS) or gas chromatography combined with mass spectrometry (GC/MS).

The skilled person will perceive the samples should be prepared in a way that it can be used appropriately for the respective analytical method. For example, various methods of absolute, or relative concentration measurement of muramic acid are known in the art. Chromatography-tandem mass spectrometry has been used for detecting trace levels of muramic acid in biological samples (Fox, 1996). Optionally, measurement of a concentrative value of muramic acid comprises an indirect measurement of a derivative obtained from sample processing, which has been described for processing of environmental samples for muramic acid analysis (Bal, 2000). Optionally, the method comprises other sample processing methods known in the art to facilitate accurate targeting measurement of a metabolite concentration value, such as addition of phosphoric acid to a sample solution to merge doublet peaks of alpha and beta anomers in high-performance liquid chromatography measurements (Watanabe, 1984).

In certain embodiments of the method according to the invention, the threshold is a level of said metabolite in a sample obtained from a healthy subject, or a representative value, such as a mean or average from a cohort of healthy samples of the same sort being analysed.

In some embodiments of the method according to the invention, the threshold for discriminating classification groups is a Fold change (FC) threshold. This refers to the fold change in metabolite abundance between melanoma and control groups, measured in test and control cohort using the same metabolite detection method as the patient sample. An example of a useful threshold according to the invention for embodiments where muramic acid concentration value is measured is provided as follows:
In first step obtaining a concentration value of muramic acid from a patient sample and a concentration value of muramic acid from a healthy control sample. Alternatively, the concentration of the muramic acid from a control sample may be replaced by a reference control value, for example, a mean or median muramic acid obtained from a cohort, for example 20, or 100 healthy control samples.

In a second step calculating a ratio value reflecting the fold-change of the muramic acid concentration level from a patient relative to controls. For example, in some embodiments, a ratio value is calculated as follows:
log2 (concentration value muramic acid of patient sample / concentration value muramic acid of control sample)

In this case, an appropriate threshold discriminating patients and healthy controls is a FC, or ratio value in the range of 1.8 to 2.2, particularly wherein the threshold is around 2.

In an assignment step, one of two classes is assigned to the subject as follows:
A first class indicating melanoma is present in the subject is assigned if the ratio is above the muramic acid ratio threshold, or a second class indicating melanoma is not present in the subject is assigned if the ratio is below the muramic acid ratio threshold value.

In particular embodiments, the metabolite concentration value is the log2FC of muramic acid compared to a control sample or value, and the threshold, if test value is higher than the FC threshold in test groups between 1.8 to 2.2, this indicates melanoma has been detected. An average or median muramic acid intensity measurement from healthy nonmelanoma patients can be used as a reference point to compare the FC. If the log2FC of muramic acid compared to a control sample or value is greater than 2 FC, the test will assign melanoma detected.

The skilled person will recognise these thresholds may be expressed differently if the data is transformed in some manner, for example, if a fold change transformation of a patient sample metabolite measure is compared to a control value with the formula log2(Control / Melanoma). In this situation, a negative FC indicates the direction of the comparison in this case, if a control group of samples is used as the reference group, a metabolite upregulated in melanoma (e.g. muramic acid) would still result in a negative log2FC when calculated as log2(Control / Melanoma).

In some embodiments of the method according to the invention, the threshold for assigning a subject to a class of melanoma detected or non-detected is a normalized cut-off threshold. This refers to the optimal classification boundary identified using a biomarker score (based on a sum of normalized metabolite intensities obtained from a cohort of melanoma patients, and a cohort of control non-melanoma patients). It is typically chosen to maximize measures of sensitivity and specificity.

In some embodiments, the measurement step may also be carried out by means of an immunoassay, using a molecular probe such as a polyclonal (Pab) or monoclonal antibody (Mab) or fragment thereof specific for a target biomarker metabolite, or a target biomarker metabolite conjugated to a hapten molecule. Antibodies specific for a metabolite can be generated by methods and screening processing known the skilled person, such as phage display, or hapten-conjugation of small molecules, animal immunisation, followed by hybridoma selection. ELISA used for the analysis of small molecules generally use a competitive type assay design (competitive ELISA) using a single MAb or PAb, or indirect competitive ELISA using a secondary labelled antibody. The method may comprise attaching either the sample, or an antibody to a solid phase, followed by incubation antibody or antigen conjugated to a detectable label, respectively. Optionally, an indirect detection method is used whereby the binding of a specific antibody for a target metabolite is measured by means of a secondary antibody conjugated to a detectable label. If two antibodies binding different epitopes of a target metabolite are available, a sandwich ELISA technique may provide higher sensitivity and specificity.

In certain embodiments where the metabolite value is determined by an enzyme immunoassay such as ELISA, the antigen (e.g. muramic acid standard or sample) in the fluid phase is immobilized on a solid phase, generally a microtiter plate comprised of rigid polystyrene, polyvinyl chloride, and polypropylene. Subsequently, the antigen is allowed to react with a molecular probe specific for the metabolite, such as a specific antibody, which is directly detected by means of a detectable label, or indirectly measured with a secondary antibody.

In particular embodiments, the detectable label used in an immunoassay is an enzyme, permitting the development of colour using a chromogenic substrate corresponds to the presence (concentration value) of the antigen. For example, horse radish peroxidase (HRP) catalyses the conversion of a chromogenic substrates including 2,2'-azino-bis(3-ethylbenzothiazoline-6-sulfonic acid) diammonium salt, 3,3',5,5'-tetramethylbenzidine, and o-phenylenediamine into coloured products, which can be detected using a microtiter plate reader.

In certain embodiments of the method according to the invention, the concentration value of the metabolite is determined using an indirect measurement assay based on enzymatic transformation of a metabolite, or degradation of metabolite to a secondary product, followed by measurement of the secondary product. Taking muramic acid as an example, muramic acid can degrade to lactic acid, which itself degrades to acetaldehyde. Acetaldehyde can be measured by means of a colorimetric assay using p-hydrozydiphenyl. In an assay using a standard curve of muramic acid, a concentration value of muramic acid can be determined by comparing a sample value to a linear relationship of muramic acid concentration and absorbance read in a microtiter plate reader at 560 nm (Hadzija, 1974). Colorimetric methods based on indirect measurement of lactate and D-lactate can also be used to detect muramic acid in clinical samples (Hoijer, 1995).

In certain embodiments of the method according to the invention, the concentration value of the metabolite is determined using an assay listed in Table 4.

### Biological samples

The method as described in the preceding section entitled *Method to detect melanoma* is designed for biological samples obtained from a subject suspected of having melanoma, or a melanoma patient for whom disease status is being tracked in a theragnostic approach. Sample acquisition from the patient is not a part of the method.

The sample to which the method is applied is not particularly limited, but relates to measurement of systemic metabolites. Liquid samples such as urine, blood, serum or plasma representative of systemic metabolites may provide informative input. In particular embodiments of the method according to any aspect of the invention, the biological sample is a blood sample. In certain embodiments of the method according to any aspect of the invention, the biological sample is a plasma sample. In certain embodiments of the method according to any aspect of the invention, the biological sample is a serum sample.

### Kits according to the invention

Another aspect of the invention is a kit for detecting melanoma comprising a detection molecular probe specific for a metabolite selected from muramic acid, N-(1-deoxy-1-fructosyl)leucine, N-(1-deoxy-1-fructosyl)valine, N-(1-deoxy-1-fructosyl)methionine, 1-[(5-amino-5-carboxypentyl)amino]-1-deoxyfructose, dihydrolipoate, N-(1-deoxy-1-fructosyl)-glycine, N-(1-deoxy-1-fructosyl)-histidine, N-(1-deoxy-1-fructosyl)-leucine, distichonic acid A, hydroxypyruvate, toluene-cis-dihydrodiol, and acetyl-leucine wherein said molecular probe is conjugated to a detectable label. The kit may optionally also include positive/negative controls, and a capture agent to immobilise the metabolite on a plate or sample processing surface. The kit may optionally include a standard comprising a known concentrations of metabolites according to the invention.

Another aspect of the invention is a metabolite selected from muramic acid, N-(1-deoxy-1-fructosyl)leucine, N-(1-deoxy-1-fructosyl)valine, N-(1-deoxy-1-fructosyl)methionine, 1-[(5-amino-5-carboxypentyl)amino]-1-deoxyfructose, dihydrolipoate , N-(1-deoxy-1-fructosyl)-glycine, N-(1-deoxy-1-fructosyl)-histidine, N-(1-deoxy-1-fructosyl)-leucine, distichonic acid A, hydroxypyruvate, toluene-cis-dihydrodiol, and acetyl-leucine for the manufacture of a kit for detecting melanoma, or evaluating a cancer patient.

Another aspect of the invention is a kit comprising an ELISA assay well-plate, and a reagent comprising metabolite according to the invention, and a reagent comprising an anti-metabolite detection antibody conjugated to a chromogenic label.

### Method of Treatment or Evaluation of a cancer according to the invention

Similarly, the invention encompasses methods of treatment of cancer, comprising performing the method to detect melanoma, and performing a melanoma staging evaluation of said patient. In particular embodiments, the method comprises administering to a patient in need thereof a therapeutically effective amount of drug, or its pharmaceutically acceptable salt, as specified in detail herein.

A method of treating a cancer patient comprising performing the method to detect melanoma on a sample obtained from said patient as specified in any one of the previous aspects and embodiments of the invention described above. When an increased metabolite concentration value of the sample indicates melanoma is present, the method comprising performing a staging evaluation for melanoma.

In particular embodiments, the method of treating a cancer patient comprises stage-appropriate treatment, such as surgical removal of cancer and margin, and optionally lymph nodes for stage I and II melanoma disease. For stage II, III, and IV, the method may comprise administering an effective dose of a chemotherapeutic drug, particularly a check point inhibitor drug, or a BRAF inhibitor drug to said patient when the level of the metabolite is increased relative to healthy control sample. For stage III, and IV, the method may comprise radiation therapy, or vaccine therapy.

In particular embodiments, the method of treating a cancer patient comprises determining the level of muramic acid in a blood sample, particularly a serum or plasma sample obtained from a patient, wherein the level is determined by a method selected from HPLC or GC coupled to a suitable detection method such as, for example, UV/VIS absorption, nuclear magnetic resonance (NMR), or mass spectrometry (MS), by targeted enzyme-linked immune-sorbent assay (ELISA), or by a radio immune assay, to obtain a level of muramic acid. In particular embodiments of the method the level of the metabolite is measured by liquid chromatography combined with mass spectrometry (LC/MS) or gas chromatography combined with mass spectrometry (GC/MS).

Another aspect of the invention is pharmaceutical composition comprising a chemotherapeutic agent, particularly a checkpoint inhibitor antibody, or a BRAF or MEK inhibitory agent, for use in a method for treating malignant melanoma in a human patient having increased expression of a biomarker compared to a reference or healthy control. The method comprising assaying a blood sample from the patient (particularly a serum or plasma sample), determining if the patient has biomarker being significantly increased concentration value of muramic acid, N-(1-deoxy-1-fructosyl)leucine, N-(1-deoxy-1-fructosyl)valine, N-(1-deoxy-1-fructosyl)methionine, 1-[(5-amino-5-carboxypentyl)amino]-1-deoxyfructose, dihydrolipoate, , N-(1-deoxy-1-fructosyl)-glycine, N-(1-deoxy-1-fructosyl)-histidine, N-(1-deoxy-1-fructosyl)-leucine, distichonic acid A, hydroxypyruvate, toluene-cis-dihydrodiol,or acetyl-leucine and performing a melanoma staging evaluation, optionally evaluating a melanoma tissue sample for BRAF and/or MEK drug target gene expression, and administering a therapeutically effective amount of compound to the patient having the increased biomarker and a stage II, III, or IV melanoma, and/or expression of MEK and BRAK inhibitor agents by a melanoma sample.

Another aspect of the invention is a method for evaluating a cancer, comprising measuring a level of a metabolite selected from muramic acid, N-(1-deoxy-1-fructosyl)leucine, N-(1-deoxy-1-fructosyl)valine, N-(1-deoxy-1-fructosyl)methionine, 1-[(5-amino-5-carboxypentyl)amino]-1-deoxyfructose, dihydrolipoate, N-(1-deoxy-1-fructosyl)-glycine, N-(1-deoxy-1-fructosyl)-histidine, N-(1-deoxy-1-fructosyl)-leucine, distichonic acid A, hydroxypyruvate, toluene-cis-dihydrodiol, and/or acetyl-leucine in a sample derived from a subject; and evaluating a melanoma cancer in the subject based on the measurement results;
wherein the metabolite has a structure of C₉H₁₇NO₇, C₉H₁₇NO₇, C₁₂H₂₃NO₇, C₁₁H₂₁NO₇S, C₁₂H₂₄N₂O₇, and C₈H₁₆O₂S₂, and where the measured value of the metabolite is higher than a healthy subject reference sample or threshold indicates that a melanoma is present in the subject.

The evaluation of melanoma the subject comprises determination of a cancer stage in the subject, determination of a cancer prognosis in the subject, evaluation of tumour expression of drug targets such as BRAF, or MEK, or evaluation of the effect of a treatment on a cancer present in the subject.

### Diagnostic systems according to the invention

The invention further encompasses the use of metabolites selected from muramic acid, N-(1-deoxy-1-fructosyl)leucine, N-(1-deoxy-1-fructosyl)valine, N-(1-deoxy-1-fructosyl)methionine, 1-[(5-amino-5-carboxypentyl)amino]-1-deoxyfructose, dihydrolipoate, N-(1-deoxy-1-fructosyl)-glycine, N-(1-deoxy-1-fructosyl)-histidine, N-(1-deoxy-1-fructosyl)-leucine, distichonic acid A, hydroxypyruvate, toluene-cis-dihydrodiol, and/or acetyl-leucine identified herein for use in the manufacture of a kit for the detection of melanoma in a sample obtained from a subject.

The invention further encompasses the use of antibodies binding specifically to a metabolite selected from muramic acid, N-(1-deoxy-1-fructosyl)leucine, N-(1-deoxy-1-fructosyl)valine, N-(1-deoxy-1-fructosyl)methionine, 1-[(5-amino-5-carboxypentyl)amino]-1-deoxyfructose, dihydrolipoate , N-(1-deoxy-1-fructosyl)-glycine, N-(1-deoxy-1-fructosyl)-histidine, N-(1-deoxy-1-fructosyl)-leucine, distichonic acid A, hydroxypyruvate, toluene-cis-dihydrodiol, and/or acetyl-leucine identified herein for use in the manufacture of a kit for the detection of melanoma in a sample obtained from a subject.

### Biomarker composition

Another aspect of the invention is a biomarker composition which comprises a molecular probe for detecting the presence of biomarker selected from muramic acid, N-(1-deoxy-1-fructosyl)leucine, N-(1-deoxy-1-fructosyl)valine, N-(1-deoxy-1-fructosyl)methionine, 1-[(5-amino-5-carboxypentyl)amino]-1-deoxyfructose, dihydrolipoate, , N-(1-deoxy-1-fructosyl)-glycine, N-(1-deoxy-1-fructosyl)-histidine, N-(1-deoxy-1-fructosyl)-leucine, distichonic acid A, hydroxypyruvate, toluene-cis-dihydrodiol, or acetyl-leucine or determining the expression level of said metabolites in blood, for example, by mass spectrometry, NMR, or ELISA.

### Method of providing information for diagnosis

Another aspect of the invention is a method for providing diagnostic information about melanoma, the method comprising detecting the presence of biomarker selected from muramic acid, N-(1-deoxy-1-fructosyl)leucine, N-(1-deoxy-1-fructosyl)valine, N-(1-deoxy-1-fructosyl)methionine, 1-[(5-amino-5-carboxypentyl)amino]-1-deoxyfructose, dihydrolipoate, or , N-(1-deoxy-1-fructosyl)-glycine, N-(1-deoxy-1-fructosyl)-histidine, N-(1-deoxy-1-fructosyl)-leucine, distichonic acid A, hydroxypyruvate, toluene-cis-dihydrodiol, or acetyl-leucine in an isolated sample obtained from a patient or determining the level of expression of said biomarker in an isolated sample obtained from a patient.

Wherever alternatives for single separable features are laid out herein as "embodiments", it is to be understood that such alternatives may be combined freely to form discrete embodiments of the invention disclosed herein. Thus, any of the alternative embodiments for a metabolite may be combined with any of the alternative embodiments sample type, or any detection method, and these combinations may be combined with diagnostic method or patient stratification method mentioned herein.

The invention is further illustrated by the following examples and figures, from which further embodiments and advantages can be drawn. These examples are meant to illustrate the invention but not to limit its scope.

### Description of the Figures

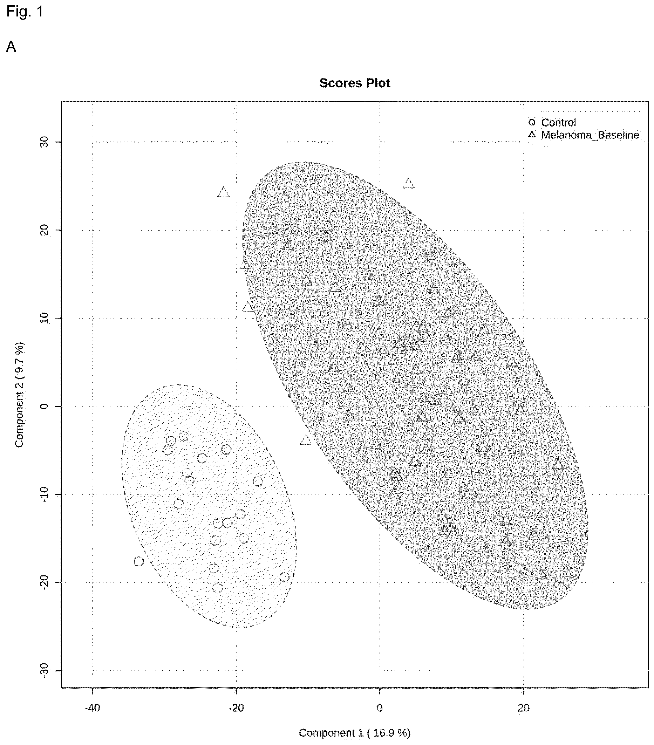

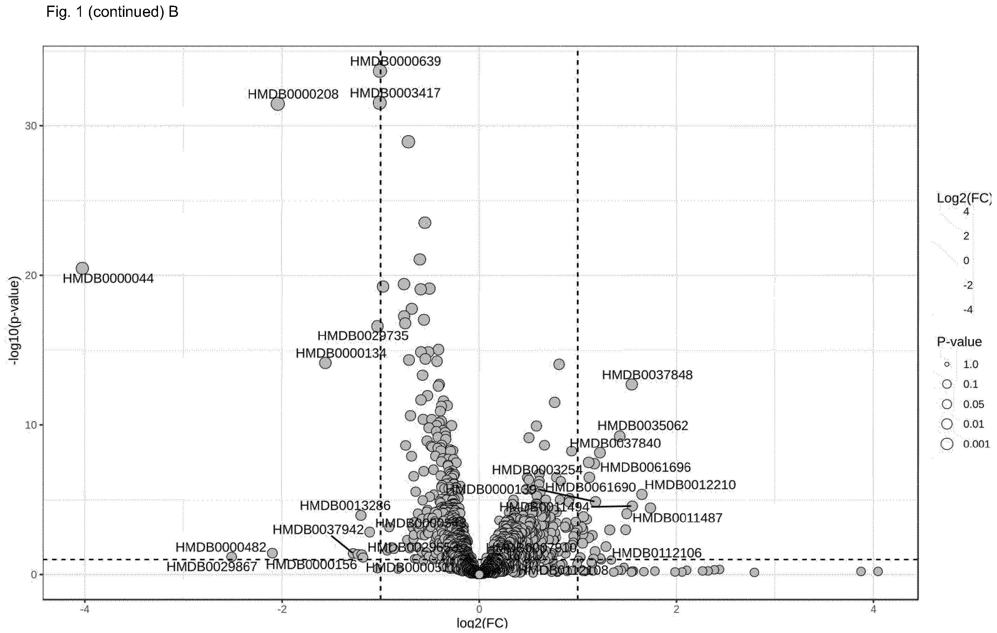

Fig. 1 shows multivariate analysis and metabolite profiling demonstrates striking differences in the serum metabolome between healthy control individuals and patients with melanoma. (A) Partial Least Squares Discriminant Analysis (PLS-DA) showing a clear separation between 87 melanoma patients and 18 healthy controls based on the 1466 metabolites detected. (B) Volcano plot showing the log2 fold change on the x-axis and -log 10 p-value on the y-axis.

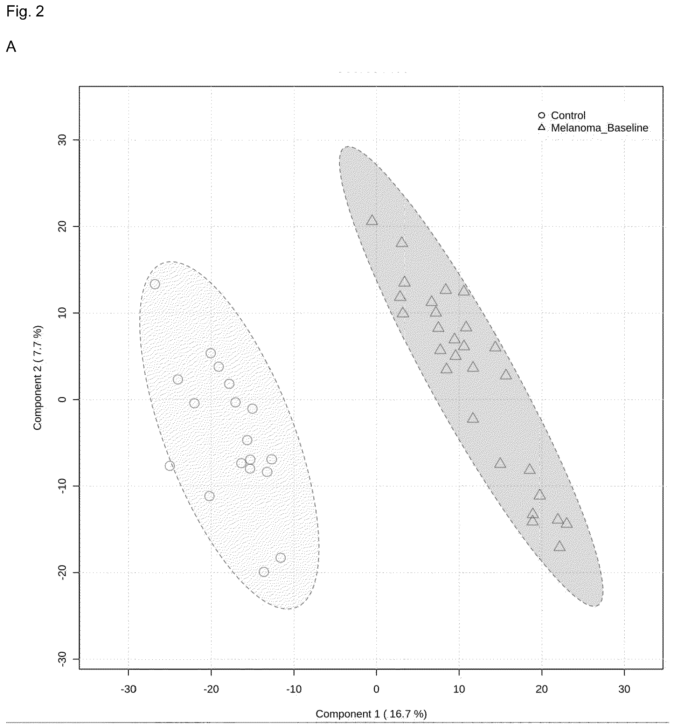

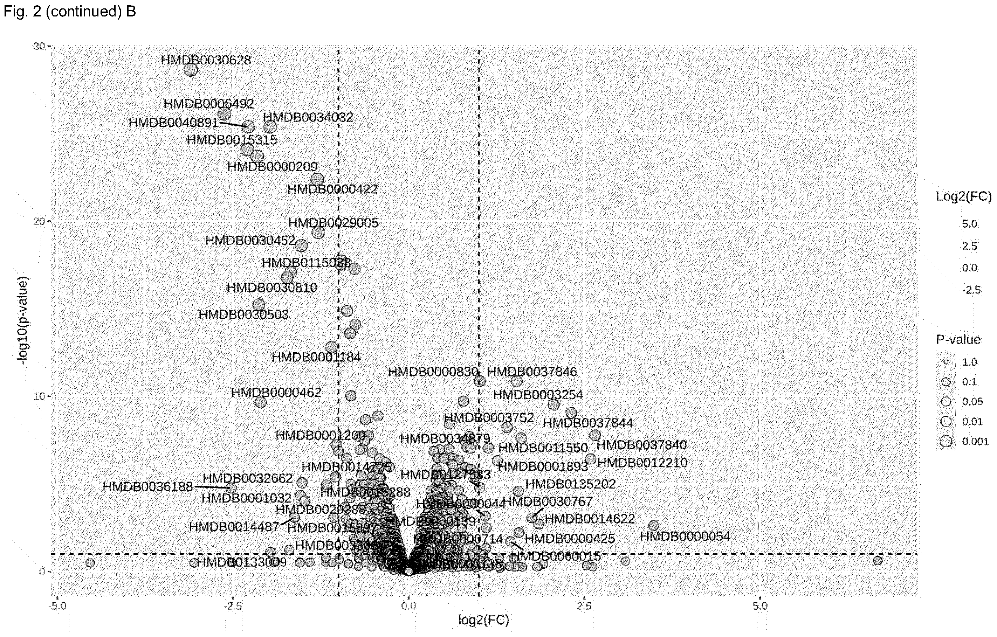

Fig. 2 shows serum metabolome data from a validation cohort consisting of 37 patients with melanoma confirm the difference between healthy control individuals and melanoma patients. (A) Partial Least Squares Discriminant Analysis (PLS-DA) showing a clear separation between 37 melanoma patients and 18 healthy controls based on the 1466 metabolites detected. (B). Volcano plot showing the log2 fold change on the x-axis and -log 10 p-value on the y-axis.
Fig. 3 shows serum metabolomics analysis identify potential serum biomarkers for the diagnosis of patients with melanoma. (A-F) Intensity levels of the top 6 selected metabolites [Muramic acid, N-(1-Deoxy-1-fructosyl)leucine,N-(1-Deoxy-1-fructosyl)-valine,N-(1-Deoxy-1-fructosyl)methionine, 1-[(5-Amino-5-carboxypentyl)amino]-1-deoxyfructose, Dihydrolipoate] and their corresponding ROC curves. (G) Specificity of muramic acid elevation in melanoma, compared to ulcerative colitis, or Crohn's disease samples.

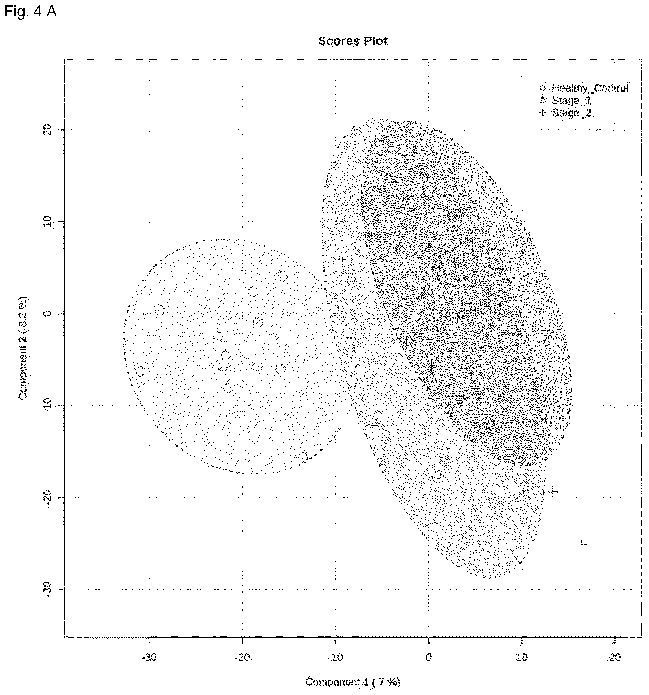

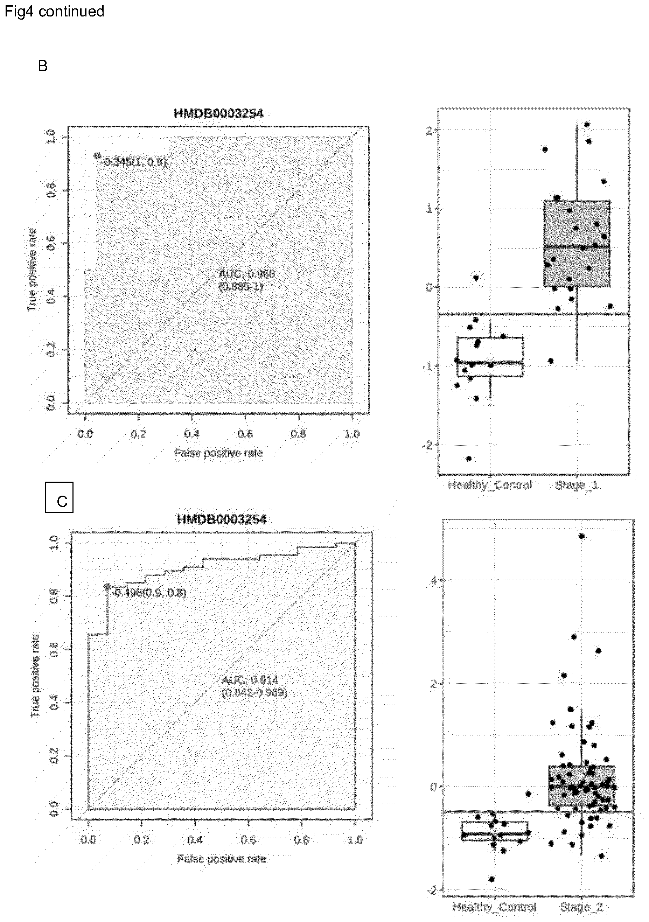

Fig. 4 shows serum metabolome data from a cohort consisting of patients with stage I and stage II melanoma further confirm the difference between healthy control individuals and melanoma patients. (A) Partial Least Squares Discriminant Analysis (PLS-DA) showing a clear separation between 22 stage I, 67 stage II melanoma patients and 14 healthy controls based on the 1466 metabolites detected. Intensity levels of Muramic acid in (B) stage I and (C) stage II melanoma patients and the corresponding ROC curves.
Fig. 5 shows how metabolite biomarkers including muramic acid, can be combined to provide a diagnostic classification. Raw metabolite data were subset to include only metabolites above 0.7. Each sample's biomarker score was computed as the sum of the normalized intensities of these metabolites. The top 10 were ranked based on the biomarker score and AUC values. A ROC curve shows the combined predictive power for the exploratory and validation cohort for (A) the overall top 10 metabolite biomarkers (B) the top amino acid markers.

### Examples

### Example 1: Untargeted serum metabolome analysis reveals a distinct metabolome composition in patients with melanoma compared to healthy individuals

To determine the complete serum metabolome composition of patients with melanoma, the inventors prospectively collected serum samples from an exploratory cohort of 87 patients with melanoma as well as from an independent validation cohort of 37 additional patients with melanoma featuring late-stage tumours. Samples were collected at baseline just before the initiation of a cancer immunotherapy. In addition, serum samples from 18 healthy control individuals in the same age range were included as a control group (Table 1).

**Table 1. Patient characteristics**

| Cohort | Disease | SE X | Patient_ Count | Mean_ Age | SD_ Age | Stage_I | Stage_II | Stage_III | Stage_IV |
|---|---|---|---|---|---|---|---|---|---|
| Exploratory | CTRL | F | 11 | 36.18 | 8.13 | NA | | | |
| Exploratory | CTRL | M | 7 | 34.14 | 6.39 | | | | |
| Exploratory | Melanoma_ baseline | F | 38 | 58.84 | 16.3 8 | 0 | 1 | 48 | 38 |
| Exploratory | Melanoma_ baseline | M | 49 | 60.27 | 12.2 9 | | | | |
| validation | CTRL | F | 11 | 36.18 | 8.13 | NA | | | |
| validation | CTRL | M | 7 | 34.14 | 6.39 | | | | |
| validation | Melanoma_ baseline | F | 14 | 62.57 | 14.3 3 | 0 | 0 | 15 | 21 |
| validation | Melanoma_ baseline | M | 23 | 67.04 | 13.8 9 | | | | |
| Early_stage | CTRL | F | 5 | 37.80 | 8.96 | NA | | | |
| Early_stage | CTRL | M | 9 | 48.78 | 15.0 1 | | | | |
| Early_stage | Melanoma_ baseline | F | 34 | 64.12 | 16.1 8 | 22 | 67 | 0 | 0 |
| Early_stage | Melanoma_ baseline | M | 55 | 67.56 | 12.1 3 | | | | |

A principal component analysis (PCA) of the exploratory cohort (87 patients with melanoma) and 18 control individuals demonstrated a clear separation between the two patient groups. Similarly, a Partial Least Squares-Discriminant Analysis (PLS-DA), visualizing the variance between samples demonstrated a clear separation between samples from healthy controls and patients with melanoma (Fig. 1A). This distinct separation indicates that the serum metabolome of patients with melanoma strongly differs from that of healthy individuals, suggesting that the serum metabolome can potentially identify patients with melanoma and thus be useful for diagnostic purposes. Out of the 1466 detected metabolites, 840 significantly differed between the two groups. Furthermore, 35 metabolites showed fold changes above 2 or below 0.5, indicating a substantially different abundance in patients with melanoma and healthy controls. Among them, 23 metabolites showed higher serum levels in patients with melanoma, including dihydrolipoate, N-(1-Deoxy-1-fructosyl)glycine, and Muramic acid, while 12 metabolites showed lower serum levels compared to healthy controls, including Ascorbate, Octanoic acid, Oxoglutarate and Fumarate (Fig. 1B and Table 2).

After demonstrating the pronounced difference between the serum metabolome of patients with melanoma and that of healthy controls, the results were confirmed in an independent validation cohort consisting of 37 additional patients with melanoma. Again, serum was collected from melanoma patients at baseline before the initial start of cancer immunotherapy, and the analysis included the same serum samples from the control cohort consisting of 18 healthy individuals. The validation cohort, corroborated previous findings from the exploratory cohort (Figs. 1+2). A clear separation was observed between the serum samples from the healthy controls and the patients with malignant melanoma, both in the PCA and PLS-DA analysis (Fig. 2A and not shown). A volcano plot of the 50 most prominent metabolites demonstrated significantly up- and downregulated metabolites were present (Fig 2B). Functional metabolome analysis of our both cohorts using an MSEA analysis showed a clear enrichment of lipids and lipid-like molecules, fatty acyls and organic acids. Pathway enrichment analysis revealed different subtypes of amino acid metabolisms as the most affected metabolic pathways in the serum of patients with melanoma (data not shown).

### Serum metabolites - clinically relevant biomarkers for the diagnosis of patients with melanoma

The inventors combined the serum metabolome data of both cohorts and aimed to identify specific metabolites that could serve as clinically relevant biomarkers for the diagnosis of melanoma. When combining the results of all serum analyses and presenting these data within a new heat-map showing the 50 most prominent metabolites of both cohorts, the inventors confirmed 100% accuracy of clustering separation between samples from the patients with melanoma and the samples of the healthy controls (not shown). By performing Receiver Operating Characteristic (ROC) curve and area under the curve (AUC) analysis, the performance of the top metabolites as biomarkers was evaluated. With this approach, numerous metabolites showed promising AUC curve values over 0.7 (Table 2). The inventors further analysed six serum metabolites that might serve as powerful biomarkers for the diagnosis of melanoma in clinical practice, namely muramic acid, N-(1-deoxy-1-fructosyl)leucine, N-(1-deoxy-1-fructosyl)valine, N-(1-deoxy-1-fructosyl)methionine, 1-[(5-amino-5-carboxypentyl)amino]-1-deoxyfructose and dihydrolipoate exhibited an area under the curve (AUC) of at least 0.9544 in both the exploratory and the validation cohort (Figs. 3A-F). The best performance was detected for the bacterial wall component muramic acid, which featured AUCs of 0.9936 and 1.0 for the diagnosis of melanoma in the exploratory cohort and in the validation cohort, respectively. Notably, all six biomarkers were strongly upregulated in the serum of patients with melanoma when compared to healthy controls in both, the exploratory as well as the validation cohort. To verify that the muramic acid elevation in melanoma patients is specific to melanoma and not solely attributed to inflammation, the inventors also tested the measurement in IBD patients, and it is indeed specific to melanoma patients (Fig. 3G).

The selected biomarkers exhibited a notable AUC cutoff of 0.9, indicating their high discriminatory capacity in distinguishing melanoma cases. These biomarkers were overlapped across both, the exploratory and validation cohorts, affirming their consistent identification and reliability in differentiating melanoma cases. Moreover, quantitative analysis show-cased a uniform intensity measurement for these biomarkers across various samples, emphasizing their stability and potential as reliable indicators for melanoma detection or prognosis.

However, there was no obvious contribution of clinical characteristics, such as tumour stage, BRAF mutation status, onset of immune-related adverse events or therapy response to the clustering (not shown). Further, the tested biomarkers were not affected by the age of the respective patients (not shown).

**Table 2: Receiver operating curve AUC and associated p value (of which tests) for the exploratory (Explore) and validation (Validate) cohorts. ID shows human metabolite database identifier code.**

| **ID** | **Explore AUC** | **Explore Pval** | **Validate AUC** | **Validate Pval** | **Metabolite** |
|---|---|---|---|---|---|
| HMDB0037848 | 1,00 | 3,6641E-15 | 1,00 | 1,3007E-09 | N-(1-Deoxy-1-fructosyl)-glycine |
| HMDB0003254 | 0,99 | 1,5975E-09 | 1,00 | 4,9231E-12 | Muramic acid |
| HMDB0037839 | 0,99 | 1,4456E-16 | 0,92 | 0,00020258 | N-(1-Deoxy-1-fructosyl)-histidine |
| HMDB0037841 | 0,98 | 8,5464E-11 | 0,95 | 2,6166E-07 | N-(1-Deoxy-1-fructosyl)-methionine |
| HMDB0037840 | 0,98 | 3,2791E-10 | 1,00 | 3,3297E-10 | N-(1-Deoxy-1-fructosyl)-leucine |
| HMDB0038752 | 0,97 | 2,4734E-11 | 0,93 | 1,4416E-07 | Distichonic acid A |
| HMDB0000691 | 0,97 | 3,4444E-22 | 0,94 | 1,6455E-07 | Hydroxypyruvate |
| HMDB0037844 | 0,97 | 2,4629E-10 | 1,00 | 1,5083E-11 | N-(1-Deoxy-1-fructosyl)-valine |
| HMDB0012210 | 0,96 | 3,6633E-07 | 1,00 | 1,3874E-08 | Dihydrolipoate |
| HMDB0029582 | 0,96 | 3,5991E-15 | 0,79 | 0,00104839 | Toluene-cis -dihydrodiol |
| HMDB0061696 | 0,96 | 1,9971E-09 | 0,87 | 1,8763E-05 | 1-Stearoylglycero-phosphoinositol |
| HMDB0061690 | 0,96 | 1,9995E-08 | 0,92 | 3,3326E-08 | 1-Arachidonoyl-glycero-phosphoinositol |
| HMDB0000139 | 0,94 | 1,3046E-06 | 0,96 | 0,00067522 | D-Glyceric acid |
| HMDB0000209 | 0,94 | 4,682E-12 | 1,00 | 7,8455E-27 | Phenyl acetate |
| HMDB0000701 | 0,94 | 3,0129E-08 | 1,00 | 2,6429E-09 | Acetyl-Leu |
| HMDB0011494 | 0,93 | 2,7764E-06 | 0,85 | 2,7748E-05 | LysoPE(0:0/22:5(4Z, 7Z, 1 OZ, 13 Z,16Z)) |
| HMDB0129377 | 0,93 | 1,3138E-10 | 0,82 | 5,6915E-05 | 2-(2H-1,3-benzodioxol-5-yl) -2-oxoacetic acid |
| HMDB0000709 | 0,93 | 6,0462E-13 | 0,97 | 1,3539E-12 | L-Cysteinylglycine disulfide |
| HMDB0031532 | 0,92 | 2,3768E-07 | 0,70 | 0,09685518 | 1-Methyl-1, 3-cyclohexadiene |
| HMDB0011487 | 0,92 | 4,1619E-06 | 0,77 | 0,0020786 | LysoPE(0:0/20:4(5Z,8Z,11 Z,14Z)) |
| HMDB0000462 | 0,92 | 1,6489E-08 | 0,98 | 3,4919E-12 | Allantoin |
| HMDB0000530 | 0,91 | 6,7445E-09 | 0,93 | 0,000347 | 6-Ketoestriol |
| HMDB0061695 | 0,90 | 4,1256E-06 | 0,74 | 0,02240786 | 1-Palmitoylglycero-phosphoinositol |
| HMDB0041274 | 0,90 | 0,00013737 | 0,74 | 0,00664661 | Phenylethyl primeveroside |
| HMDB0032662 | 0,89 | 1,5232E-08 | 0,93 | 4,792E-07 | (S)-9-Hydroxy-10-undecenoic acid |
| HMDB0029388 | 0,88 | 0,00014259 | 0,89 | 9,5432E-06 | Cucurbic acid |
| HMDB0033191 | 0,88 | 7,6803E-09 | 0,75 | 0,00546402 | 2,2,4,4,-Tetramethyl-6-(1-oxopropyl)-1,3,5-cyclohexanetrione |
| HMDB0000572 | 0,88 | 0,00024129 | 0,92 | 7,4171E-07 | Desmosine |
| HMDB0037683 | 0,88 | 2,2949E-05 | 0,77 | 0,40626283 | Ethyl vanillin isobutyrate |
| HMDB0004951 | 0,88 | 3,4333E-06 | 0,80 | 0,00051661 | C20 Cer |
| HMDB0001878 | 0,87 | 0,00014318 | 0,78 | 0,00650502 | Thymol |
| HMDB0035665 | 0,87 | 0,00703606 | 0,84 | 1,7265E-05 | L-1,2,3,4-Tetrahydro-beta-carboline-3-carboxylic acid |
| HMDB0034462 | 0,87 | 5,4922E-08 | 0,86 | 0,00093015 | Heptyl 4 -hydroxybenzoate |
| HMDB0029721 | 0,87 | 0,00138302 | 0,78 | 0,00170217 | 2,3,5-Trimethylfuran |
| HMDB0013105 | 0,87 | 5,5174E-07 | 0,86 | 0,00030037 | C10:2 |
| HMDB0035486 | 0,86 | 5,3886E-05 | 0,87 | 0,00037731 | 6-Caffeoylsucrose |
| HMDB0001539 | 0,86 | 9,8878E-05 | 0,81 | 0,000444 | Dimethyl-Arg |
| HMDB0000870 | 0,86 | 2,2562E-08 | 0,81 | 0,00030213 | Histamine |
| HMDB0028919 | 0,86 | 0,02208108 | 0,80 | 0,00022724 | Isoleucyl-Tyrosine |
| HMDB0000339 | 0,85 | 9,1354E-07 | 1,00 | 2,8616E-12 | 2-Methylbutyrylglycine |
| HMDB0004952 | 0,85 | 1,4362E-05 | 0,76 | 0,00128691 | C22 Cer |
| HMDB0032797 | 0,85 | 5,7964E-08 | 0,88 | 1,7675E-06 | Jasmonic acid |
| HMDB0013067 | 0,85 | 1,6146E-05 | 0,91 | 0,05774827 | Salsoline-1-carboxylate |
| HMDB0013286 | 0,84 | 1,5896E-05 | 0,74 | 0,02646488 | N-Undecanoylglycine |
| HMDB0048343 | 0,84 | 3,973E-05 | 0,73 | 0,01586137 | TG(14:1(9Z)/20:5(5Z,8Z,11Z,14 Z,17Z)/22:6(4Z,7Z,10Z,13Z,16Z ,19Z)) |
| HMDB0029867 | 0,84 | 0,03082104 | 0,88 | 0,16789434 | Isoelemicin |
| HMDB0000950 | 0,83 | 6,2401E-05 | 0,72 | 0,00748824 | Cer(d18:1/23:0) |
| HMDB0030984 | 0,82 | 4,4885E-05 | 0,72 | 0,0073886 | 5-Hexyltetrahydro-2-oxo-3-furancarboxylic acid |
| HMDB0000332 | 0,80 | 0,00019406 | 0,93 | 2,3402E-05 | 18-Oxocortisol |
| HMDB0034168 | 0,80 | 0,0001205 | 1,00 | 1,3638E-17 | Toluene |
| HMDB0001999 | 0,80 | 0,00094067 | 0,73 | 0,00579637 | Eicosapentaenoic acid |
| HMDB0035388 | 0,80 | 0,00061707 | 0,73 | 0,02077097 | (24E)-3alpha,15alpha-Diacetoxy-23-oxo-7,9(11),24-lanostatrien-26-oic acid |
| HMDB0038140 | 0,79 | 0,00066386 | 0,74 | 0,02839292 | Santene |
| HMDB0004957 | 0,78 | 0,00095728 | 0,80 | 0,00053979 | Ceramide (d18:1/25:0) |
| HMDB0003822 | 0,78 | 0,00069697 | 0,79 | 0,00108256 | Cholesteryl acetate |
| HMDB0006709 | 0,77 | 0,04952937 | 0,88 | 3,5176E-06 | Ubiquinone-2 |
| HMDB0032353 | 0,77 | 0,00080284 | 0,91 | 9,9506E-05 | Isopropyl citrate |
| HMDB0011775 | 0,77 | 0,00101035 | 0,80 | 0,00284779 | Cer(d18:1/22:1(13Z)) |
| HMDB0001424 | 0,76 | 0,00038018 | 0,71 | 0,00378275 | 3-Methyldioxyindole |
| HMDB0003905 | 0,76 | 0,00295514 | 0,82 | 2,5974E-05 | N-Acetylimidazole |
| HMDB0002409 | 0,76 | 0,19908392 | 0,79 | 0,06833727 | N-[(3a,5b,7b)-7-hydroxy-24-oxo-3-(sulfooxy)cholan-24-yl]-Glycine |
| HMDB0032306 | 0,76 | 0,00095189 | 0,90 | 3,5199E-06 | 4-Heptenal diethyl acetal |
| HMDB0000402 | 0,76 | 0,01207153 | 0,83 | 0,00025598 | 3-Propylmalate |
| HMDB0015496 | 0,76 | 0,056933 | 0,78 | 0,00353351 | Amrinone |
| HMDB0014018 | 0,76 | 0,00286596 | 0,72 | 0,01365491 | 4-Hydroxypropofol |
| HMDB0030605 | 0,75 | 0,89576233 | 0,81 | 0,00022044 | 1,5-Dihydroxy-3-methoxy-2-prenylxanthone |
| HMDB0030081 | 0,75 | 0,00035278 | 0,75 | 0,24186753 | Carissanol |
| HMDB0030987 | 0,74 | 0,00022803 | 0,85 | 4,3676E-05 | 2-Carboxy-4-dodecanolide |
| HMDB0031844 | 0,74 | 0,0059063 | 0,77 | 0,01553118 | Trimethylpyrazine |
| HMDB0000122 | 0,74 | 0,00308 | 0,83 | 9,312E-05 | Hexose |
| HMDB0000482 | 0,73 | 0,0152002 | 0,75 | 0,20183079 | Octanoic acid |
| HMDB0030762 | 0,73 | 0,50848485 | 0,98 | 2,7822E-09 | Ethyl trans-p-methoxycinnamate |
| HMDB0030502 | 0,73 | 0,05950274 | 0,89 | 0,40171726 | (2S,4S,6S)-2-[2-(4-Hydroxy-3-meyhoxyphenyl)ethyl] tetrahydro-6-(4,5-dihydroxy-3-methoxyphenyl)-2H-pyran-4-ol |
| HMDB0003609 | 0,72 | 0,00214023 | 0,92 | 2,3472E-08 | Dehydroalanine |
| HMDB0031173 | 0,72 | 0,00628914 | 0,84 | 5,8084E-05 | 6-Hydroxy-1H-indole-3-acetamide |
| HMDB0012150 | 0,71 | 0,59403843 | 0,76 | 0,27436263 | 2-Keto-6-acetamidocaproate |
| HMDB0013854 | 0,71 | 0,54171954 | 0,86 | 0,00206928 | N4-Acetylsulfamethoxazole |
| HMDB0000510 | 0,71 | 0,01286765 | 0,90 | 3,8078E-05 | Aminoadipate |
| HMDB0000386 | 0,71 | 0,00124181 | 0,73 | 0,08311256 | 3b,16a-Dihydroxyandrostenone sulfate |
| HMDB0002430 | 0,70 | 0,04016433 | 0,76 | 0,01574227 | (3a, 5b, 7a)-23-Carboxy-7-hydroxy-24-norcholan-3-yl-b-D-Glucopyranosiduronic acid |

### Expanding Diagnostic Biomarkers: Serum Metabolites Validate Melanoma Across All Stages

Early diagnosis of melanoma is particularly challenging. The inventors broadened the study to validate the biomarker aspect of the identified molecules in early stage melanoma patients: 22 patients with stage 1 melanoma, 67 patients with stage 2 melanoma, and 14 control subjects (Table 1). The metabolomics profile of early-stage melanoma patients again differs from that of control individuals, as demonstrated by both, PLSDA and hierarchical clustering of altered metabolites (Figs. 4A+B). Subsequently, the inventors analysed the enriched metabolites and discovered that they were comparable to those in later stages, with carboxylic acids, fatty acyls, and organooxygen compounds identified as the most highly enriched metabolite groups . Furthermore, amino acid metabolism emerged as the primary pathway category enriched in our pathway analysis, suggesting that this metabolic process plays a role in disease progression from its early stages. Finally, the inventors assessed the AUC of our lead biomarker candidate, namely muramic acid, and found that it features an AUC of 0.964 in patients with stage I as well as of 0.908 in patients with stage II melanoma (Fig. 4B).

### Summary

The Examples herein uncovered potentially clinically relevant biomarkers for the diagnosis of melanoma using untargeted metabolomics to generate serum metabolite profiles of two independent cohorts of melanoma patients and revealed significant alterations in lipid metabolism, organic acid pathways, and amino acid synthesis metabolic pathways. These pathways correspond with known pathophysiological mechanisms involved in the development of melanoma and were consistently modified across all cohorts.

On the clinical level, the Examples provide evidence for a number of highly promising an, the exploratory and validation cohort, at least six of metabolites biomarkers are able to accurately diagnose melanoma in a clinical setting. Muramic acid in particular represents a very promising accuracy across all melanoma stages. Considering their AUC, they might be clearly superior to existing biomarkers such as S-100B or lactate dehydrogenase (LDH) in this regard. The discriminative ability of serum S100B at identifying disease relapse [pooled Area Under the ROC (AUROC) 78.64 (95% CI 70.28; 87.01)] was significantly greater than the discriminative ability of serum LDH [AUROC 64.41 (95% CI 56.05; 7278)] (p=0.013). Both are far lower than metabolite biomarkers described herein (Janka, 2021).

The claimed specific serum metabolites may serve as novel tumour markers for the diagnosis of melanoma, or further aspects of disease monitoring and patient care, such as disease recurrence.

### Example 2: Materials and methods

### Patient Recruitment

Ethical approval for collecting serum samples as well as clinical characteristics from patients with melanoma and healthy control individuals after providing written informed consent was received by the Cantonal Ethics Committee of the Canton Zürich (EK647-PB_2018-00194 and EK-1755-PB_2019-00169). Further use of the biosamples and clinical data for the specific project was granted by the Cantonal Ethics Committee of the Canton Zürich via approval KEK-ZH 2019-01337. Patients at University Hospital Zurich (USZ) with melanoma diagnosed with Union for International Cancer Control (UICC) stages I-IV and a minimum of 18 years of age were selected. After obtaining written informed consent from the patients, respective specimens and clinical data were collected. Additionally, overall, 32 control individuals were recruited from the outpatient clinic of the Department of Gastroenterology and Hepatology, University Hospital Zurich.

### Metabolomics

Blood was collected in non-coated vacutainers, and then centrifuged at 2000 g for 10 minutes at **RT.** Subsequently obtained serum was snap frozen and stored at -80°C until further processing. Metabolomics analysis was carried out by flow-injection mass spectrometry on Agilent QTOF 6550 as described previously by Fuhrer *et al.,* (Fuhrer, 2011), and we used MetaboAnalystR package version 4.0.0 for the statistical analysis (Pang, 2020). The normalized intensities by the median for each sample were mean-centred and divided by the standard deviation of each variable of all annotated features. Significance analysis was done by heteroscedastic (two tail, unequal variance) t-test. In addition, P-values were adjusted according to the false discovery rate. Partial Least Squares - Discriminant Analysis was used to visualize sample variance. Heatmap visually presented the hierarchical clustering using the Euclidean method for distance calculation and Ward's linkage for clustering. Metabolite set enrichment analysis (MSEA) was carried out against the "main" data set, and a generalized linear model was used during the quantitative enrichment analysis. The correlation between the significant metabolites and enriched pathways was conducted using the corrr package in R and the sanky plot was generated using (networkD3) package. Classical Receiver Operating Characteristic (ROC) curve analysis was performed using the pROC package in R to assess the efficacy of the selected species as a biomarker for discriminating between melanoma patients and healthy donors.

### Example 3: Representative example of clinical sample treatment according to the invention

In addition to targeted, or non-targeted metabolic analysis using mass spectrometry, various methods of analysing a patient sample are known to obtain concentration values of metabolites which can inform on the presence or absence of melanoma, such as a colorimetric assay or ELISA.
Table 3 lists commercially available kits providing sensitive measurements of metabolite biomarkers listed in Table 2.
Table 3. Kits for obtaining metabolite concentration values

| **Metabolite Name** | **Type of Kit Available** | **Sample Type (Blood Compatibility)** | **Manufacturer/Supplier** |
|---|---|---|---|
| **Eicosapentaenoic Acid (EPA)** | ELISA (Competitive Colorimetric) | Serum, Plasma | Biomatik |
| **Docosahexaenoic Acid (DHA)** | ELISA (Competitive) | Serum, Plasma | Abbexa |
| **Arachidonic Acid (AA)** | ELISA (Competitive) | Serum, Plasma | Biomatik |
| **Lysophosphatidylethanola mine (LysoPE)** | LC-MS/MS Targeted Kit (metabolomics) | Plasma (validated for serum/plasma) | Biocrates |
| **Ceramide (total)** | ELISA (Competitive) | Serum, Plasma | Antibodies.com |
| **Kynurenine** | ELISA (Competitive) | Plasma, Serum | ImmuSmol |
| **Kynurenic Acid** | ELISA (Competitive) | Serum | ImmuSmol |
| **Quinolinic Acid** | ELISA (Competitive) | Serum, Plasma | ImmuSmol |
| **3-Methylhistidine (3-MH)** | ELISA (Competitive) | Serum, Plasma | Abbexa |
| **Asymmetric Dimethylarginine (ADMA)** | ELISA (Competitive) | Serum, Plasma | Enzo Life Sciences |
| **Symmetric Dimethylarginine (SDMA)** | ELISA (Competitive) | Serum, EDTA-Plasma | Abcam |
| **Indoxyl Sulfate** | ELISA (Competitive) | Plasma, Serum | LSBio |
| **Trimethylamine N-oxide (TMAO)** | ELISA (Sandwich) | Serum, Plasma | AFG Scientific |

### Example 4: Combination biomarkers

Multiple biomarkers can be combined to provide a robust diagnostic system if included in a classification algorithm. The inventors explored whether metaboliten concentration values for multiple metabolites can combined to classify patient and healthy samples. In brief, the raw metabolite data were subset to include only metabolites above 0.7. Each sample's biomarker score was computed as the sum of the normalized intensities of these metabolites. The top 10 (N-(1-deoxy-1-fructosyl)-glycine, muramic acid, N-(1-deoxy-1-fructosyl)-histidine, N-(1-deoxy-1-fructosyl)-methionine, N-(1-deoxy-1-fructosyl)-leucine, distichonic acid A, hydroxypyruvate, N-(1-deoxy-1-fructosyl)-valine, dihydrolipoate, toluene-cis-dihydrodiol) refers to the most predictive metabolites ranked based on the biomarker score and AUC values, which when combined could effectively classify melanoma samples and control with high sensitivity and specificity. Likewise, top 6 amino acid metabolite ((N-(1-Deoxy-1-fructosyl)-glycine, N-(1-Deoxy-1-fructosyl)-histidine, N-(1-Deoxy-1-fructosyl)-methionine, N-(1-Deoxy-1-fructosyl)-leucine, Hydroxypyruvate, N-(1-Deoxy-1-fructosyl)-valine, Acetyl-Leucine) concentration values could be combined to provide effective patient and control classification (Fig. 5).

### Cited references:

D. C. Deacon, et al. Front Med (Lausanne) 8, 642380 (2021).
A. W. L. Bayci et al. Metabolomics 14, 105 (2018).
D. Baade et al., Int J Cancer 147, 1391-1396 (2020).
B. Weide et al., Br J Cancer 107, 422-428 (2012).
J. E. Gershenwald et al., CA Cancer J Clin 67, 472-492 (2017).
D. D. Weber et al., Cancers (Basel) 13, (2021).
N. J. Taylor et al., PLoS One 15, e0240849 (2020).
J. Du et al., Nature communications 11, 4830 (2020).
H. Y. Kim et al., Sci Rep 7, 8864 (2017).
X. Shi et al., Sci Adv 3, eaao5268 (2017).
A. E. Frankel et al., Neoplasia 19, 848-855 (2017).
A. Bui, et al., Methods Mol Biol 2265, 461-474 (2021).
G. Vandoni et al., Int J Mol Sci 24 (2023).
T. Fuhrer et al., Anal Chem 83 7074-7080 (2011).
Z. Pang, J. et al. Metabolites 10 (2020).
A. Fox, et al., Infection and Immunity 64(9):3911 (1996).
Bal K, L Larsson J Chrom B 738(1):57 (2000).
Watanabe N, J. Chromatogr. 316 (1984).
Hadzija O., Analytical Biochem. 60(2): 512 (1974).
Hoijer M. A. et al. Infection and Immunity 63: 1652 (1995).
Janka et al. Front Oncol. 7:11:772165 (2021).

All scientific publications and patent documents cited in the present specification are incorporated by reference herein.

## Claims

1. A method to detect melanoma in a subject, comprising a step of:
i) measuring a concentration value of at least one metabolite in a sample having been obtained from said subject, wherein the metabolite is selected from the list consisting of:
- muramic acid,
- N-(1-deoxy-1-fructosyl)-leucine,
- N-(1-deoxy-1-fructosyl)-valine,
- N-(1-deoxy-1-fructosyl)-methionine,
- 1-[(5-amino-5-carboxypentyl)-amino]-1-deoxyfructose, and
- dihydrolipoate,
- N-(1-deoxy-1-fructosyl)-glycine,
- N-(1-deoxy-1-fructosyl)-histidine,
- N-(1-deoxy-1-fructosyl)-leucine,
- distichonic acid A,
- hydroxypyruvate,
- toluene-cis-dihydrodiol
- acetyl-leucine.

2. The method according to claim 1, further comprising the steps of:
ii) comparing the concentration value to a threshold or a reference sample;
iii) assigning one of two classes to the subject, wherein
- a first class indicating melanoma is present in the subject is assigned if the concentration value is increased relative to the threshold or reference sample, or
- a second class indicating melanoma is not present in the subject is assigned if the concentration value is decreased relative to the threshold or reference sample.

3. The method according to claim 1 or 2, wherein the metabolite is muramic acid (C₉H₁₇NO₇), particularly wherein muramic acid is the only metabolite for which the concentration value is determined.

4. The method according to any one of the preceding claims, where the method comprises measuring at least 2, 3, 4, 5, 6, 7, 8, 9, or 10 metabolite concentration values.

5. The method according to any one of the preceding claims, wherein the concentration value of the metabolite is measured by a method selected from the group consisting of UV/VIS, nuclear magnetic resonance, or mass spectrometry, targeted enzyme-linked immune-sorbent assay, radio immune assay.

6. The method according to any one of the preceding claims, wherein the level of the metabolite is measured by liquid chromatography mass spectrometry (LC/MS).

7. The method according to any one of the preceding claims, wherein the threshold is a concentration value of said metabolite in a healthy control sample, or a mean, or median concentration value obtained from a cohort of healthy control samples.

8. The method according to any one of the preceding claims, wherein the method comprises:
a) obtaining a concentration value of muramic acid from a patient sample and a concentration value of muramic acid from a healthy control sample,
b) calculating a ratio value, wherein the ratio value is log2 (concentration value muramic acid of patient sample / concentration value muramic acid of control sample), and
c) assigning one of two classes to the subject as follows:
- a first class indicating melanoma is present in the subject is assigned if the ratio calculated in b) is above a threshold, or
- a second class indicating melanoma is not present in the subject is assigned if the ratio calculated in b) is below a threshold value.
wherein the threshold is a ratio value in the range of 1.8 to 2.2, particularly wherein threshold is a ratio value 1.9 to 2.1, more particularly wherein the threshold value is 2.

9. The method according to any one of the preceding claims wherein the sample is a blood sample.

10. The method according to any one of the preceding claims wherein the sample is a plasma or serum sample.
